# EUROPEAN PATENT APPLICATION

(11) **EP 2 813 154 A1**
(43) Date of publication of application: **17.12.2014**
(21) Application number: 12859587.3
(22) Date of filing: 21.12.2012
(51) Int. Cl.: A41D 13/00, A61F 5/00

(54) **TENSEGRITY-BASED GARMENT FOR OPTIMISING HUMAN POSTURE AND MOTION**

(30) Priority: 23.12.2011 BR PI1106236; 19.12.2012 BR 102012032478
(71) Applicant: Universidade Federal De Minas Gerais - UFMG, 31270-901 Belo Horizonte - MG (BR)
(72) Inventor: TEIXEIRA DA FONSECA, Sérgio, Belo Horizonte - MG, CEP: 31330-606 (BR); GUIMARÃES LOFFI, Renato, Santa Amélia Belo Horizonte - MG, CEP: 33805-550 (BR)
(74) Representative: Sarpi, Maurizio
(86) International application number: PCT/BR2012/000554
(87) International publication number: WO 2013/091061

(57) **Abstract**

The discussed matter is comprised of a biomechanical vest designed to optimize body posture and movement. Specifically, the equipment consists of a vest which has areas reinforced by a material that simulates the elastic tension usually provided by body structures. These areas of tensional reinforcement follow patterns of the musculoskeletal system architecture and aim to give appropriate support for posture and movements of children and adults with motor dysfunctions, as well as optimizing the performance of workers and sports practitioners during their activities.

## Description

The discussed subject is comprised of a biomechanical vest designed to promote optimization of body posture and movement. Specifically, the equipment consists of a vest which has areas reinforced by a material that simulates the elastic traction usually provided by body structures. These areas of tensional reinforcement follow patterns of the musculoskeletal system architecture and aim to give appropriate support for posture and movements of children and adults with motor dysfunctions, as well as optimizing the performance of workers and sports practitioners during their activities.

Proper maintenance of static posture and promotion of a dynamic control of adequate movement are fundamental conditions for the body to respond efficiently to the demands imposed to it. Therefore, it is essential that joints behave in a stable way in all situations to which they are submitted. Thus, due to its clear importance and applicability, joint stability has had prominent focus of attention and of discussion in the scientific community. Conceptually, stability can be defined as the ability of the joint to return to its original state after suffering a disturbance (Oliveira, V.C. et. al. Estabilidade articular da coluna vertebral: teorias contemporâneas e novos paradigmas Back stability: contemporary theories and new paradigms. Fisioterapia Brasil, v. 10, 2009).

The mechanical model of structural stability of constructions and bridges has been used, traditionally, for studies of stabilization mechanisms used by the neuromusculoskeletal system. Considering this model, mechanisms based on continuous compression forces have been classically proposed to describe the stability of different parts of the human body, such as the pelvis, spine and knee. However, under this perspective, there are several problems to explain joint stability of human body (Vleeming, A., et al. Relation between form and function in the sacroiliac joint. Part II: Biomechanical aspects. Spine, v.15, p.133-136, 1990a; Vleeming, A. et. al. Relation between form and function in the sacroiliac joint. Part I: Clinical anatomical aspects. Spine, v. 15, p.130-132, 1990b; Panjabi, M. M. The stabilizing system of the spine. Part II. Neutral zone and instability hypothesis. Journal of Spinal Disorder, v.5, p.390-396; discuss, 1992; Panjabi, M. M. Clinical spinal instability and low back pain. Journal of Electromyography and Kinesiology, v.13, n.4, p.371-379, 2003; Hodges, P. W. et al. Coexistence of stability and mobility in postural control: evidence from postural compensation for respiration. Experimental Brain Research, v.144, n.3, p. 293-302, 2002; Hodges, P. W. The role of the motor system in spinal pain: implications for rehabilitation of the athlete following lower back pain. Journal of Science and Medicine in Sport, v.3, n.3, p.243-253, 2004; Markolf, K., Graff-Radford, A. & Amstutz, H. In vivo knee stability. Journal of Bone and Joint Surgery, v.60-A, p. 664-675, 1978; Solomonow, M., Baratta, R.; Zhou, B. The synergistic action of the ACL and thigh muscles in maintaining joint stability. American Journal of Sports Medicine, v. 15, p. 207-213, 1987).

The stability generated by compression mechanisms would be dependent on the existence of friction forces between the joint surfaces and it would involve an excessive amount of load on these surfaces. However, there are evidences that the friction coefficients of most of the joints are extremely low to provide this kind of stability (Levin, S.M.; Theories about spinal loading. Spine, v.12, n.4, p.422-423, 1987; Levin, S.M.; Regional variation in tensile properties and biomechanical composition of the human lumbar anulus fibrosus. Spine, v.20, n.9, p.1103-1104, 1995a; Levin, S.M. The Tensegrity-Truss as a Model for Spine Mechanics: Biotensegrity. Journal of Mechanics in Medicine and Biology, v.2, n.3, p.375-388, 2002; Schmidt, T.A.; et. al. Boundary lubrication of articular cartilage: role of synovial fluid constituents. Arthritis Rheum, v. 56, n.3, p. 882-891, Mar. 2007; Wright, V.; Jonhs, R.J. Quantitative and qualitative analysis of joint stiffness in normal subjects and in patients with connective tissue diseases. Ann Rheum Dis, v. 20, p. 36-46, Mar. 1961; Such, C.H.; Unsworth, A.; Wright, V.; Dowson, D. Quantitative study of stiffness in the knee joint. Ann Rheum Dis, v. 34, n.4, p. 286-291, Ago. 1975; Whittlesey, S.N.; Robertson, D.G.E. Two-dimensional inverse dynamics. In: Robertson,D.G.E. et al. Research Methods in Biomechanics. Champaign, p.103-124, 2004).

Loads generated on joint surfaces, based on the compression mechanism, would be sufficiently high to cause degenerative processes.

Accordingly, a high prevalence of degenerative processes would be expected even at initial stages of an individual's life. However, it is observed that these processes are more prevalent in advanced ages, particularly in individuals with alterations of bone alignment, which favors compressive loads (Levin, S. M. Theories about spinal loading. Spine, v.12, n.4, p.422-423, 1987; Levin, S. M. Regional variation in tensile properties and biomechanical composition of the human lumbar anulus fibrosus. Spine, v.20, n.9, p.1103-1104, 1995a.; Levin, S. M. The Tensegrity-Truss as a Model for Spine Mechanics: Biotensegrity. J. of Mechanics in Medicine and Biology, v.2, n.3, p.375-388, 2002; Sharma L. et. al. The role of knee alignment in disease progression and functional decline in knee osteoarthritis. JAMA.; v. 286, n. 2, p.188-195, 2001; Gross K.D. et. al.. Varus foot alignment and hip conditions in older adults. Arthritis Rheum, v.56, n.9, p.2993-2998, 2007).

Besides, compression stability would be dependent on the force of gravity, in other words, position-dependent. Therefore, a joint would only be stable when an joint surface is vertically over the other, which would make the system unstable in any other position. Considering the characteristics of compression stability models, the joints would be inherently unstable. For instance, the human spine would collapse if a load of only 2 kg was applied on its top. Thus, models based on compression stability cannot explain the effective stability observed in the joints in several postures and during the movements (Levin, S. M. Theories about spinal loading. Spine, v.12, n.4, p.422-423, 1987; Levin, S. M. Regional variation in tensile properties and biomechanical composition of the human lumbar anulus fibrosus. Spine, v.20, n.9, p.1103-1104, 1995a.; Levin, S. M. The Tensegrity-Truss as a Model for Spine Mechanics: Biotensegrity. Journal of Mechanics in Medicine and Biology, v.2, n.3, p.375-388, 2002; White, A. A., Panjabi, M. M., Clinical Biomechanics of the Spine, p. 1-57. Lippincott, Philadelphia, 1978).

Considering the problems of compression models to explain the inherent stability of the musculoskeletal system, other model has been proposed. This model is based on the architectural property of tensional integrity, known as tensegrity. Tensegrity refers to an intrinsically stable system which contains a group of components under compression within a net of interconnected components under tension (traction). Tensegrity structures are stable in all directions (omnidirectional stability), due to the presence of this continuous tensional force distributed among all elements (prestress) and of an architectural organization in which the internal elements follow a totally triangular pattern (geodesic patterns) (Motro, R.; Tensegrity: The state of the art. 5th Int. Conf. on Space Structures, G.A.R. Parke and P. Disney, eds.,Telford, 2002; Sultan, C., Corless, M., & Skelton, R. E. The prestressability problem of tensegrity structures: some analytical solutions. Intern. J. of Solids and Structures, v.38, n. 30-31, p.5223-5252, 2001; Sultan, C., Corless, M., Skelton, R. E. Linear dynamics of tensegrity structures. Engineering Structures, v.24, n.6, p.671-685, 2002; Defossez, M. Shape memory effect in tensegrity structures. Mechanics Research Communications, v.30, n. 4, p.311-316, 2003).

The complex organization of the musculoskeletal system is, in fact, consistent with tensegrity structures. According to this system, bones are discontinuous compression elements, while fascias, ligaments, muscles and tendons form a continuous net of traction elements into which the bones are embedded. For instance, the fascial organization of the tissues around the lumbar spine (thoracolumbar fascia) resembles geodesic structures that obtain dynamic balance through tension forces which are distributed in all directions. Besides, studies confirm the presence of prestress in connective tissues of the musculoskeletal system at the ankle joint (Levin, S.M. The Importance of Soft Tissues for Structural Support of the Body. Spine: State of the Art Rev., v.9, 1995b; Levin, S.M. Putting the shoulder to the wheel: a new biomechanical model for the shoulder girdle. Biomedical Sci. Instrum., v.33, p.412-417, 1997a; Levin, S.M.; A different approach to the mechanics of the human pelvis: Tensegrity, Vleeming, Movement, Stability and Low back pain. Churchil Livingstone. 1997b; Souza T.R. et. al.. Prestress revealed by passive co-tension at the ankle joint. J. Biomech. v.42, n.14, p.2374-2380, 2009).

As in other tensegrity structures, internally generated forces in the musculoskeletal system or forces externally applied are immediately redistributed to different body parts to guarantee stability. Furthermore, several studies on postural adjustments to disturbances suggest that the musculoskeletal system acts globally to recover its stability and also shows responses in areas distant of those that suffer the disturbances. These facts indicate that the musculoskeletal system acts as a mean of stress propagation that, immediately, redistributes forces globally, as expected in tensegrity structures. The implication is that the musculoskeletal system architecture seems to be part of the solution and not part of the problem of stability (Neptune R. R., Zajac F.E. & Kautz S.A. Muscle force redistributes segmental power for body progression during walking. Gait Posture, v.19, n.2, p.194-205, 2004; Marsden, C. D., Merton, P.A. & Morton, H. B. Rapid postural reactions to mechanical displacement of the hand in man. In Desmedt: Motor Control Mechanisms in Health and Disease. Raven Press, p. 645-659, 1993).

The capacity of stress propagation in the musculoskeletal system seems to depend on the tissue properties such as capacity of force generation and resistance to movement (stiffness). Studies suggest being possible to alter both capacity of force generation and mechanical resistance offered by muscles to the joint movement, through a muscular training in specific positions. Experimental studies carried out with animal models demonstrate the adaptability of muscle tissue to different functional demands through modifications in stiffness, in the length-tension curve and in the muscle length (Fournier, M. et al. Is limb immobilization a model of muscle disuse? Experimental Neurology, v. 80, n.1, p. 147-156, 1983; Herbert, R. The passive mechanical properties of muscle and their adaptations to altered patterns of use. The Australian Journal of Physical Therapy, v. 34, n.3, p. 141-149, 1988).

When maintained in shortened position, the muscles suffer a loss of up to 40% in the number of sarcomeres in series, associated with reduction of length and increase of muscular stiffness (Tabary, J.C. et al. Physiological and structural changes in the cat's soleus muscle due to immobilization at different lengths by plaster casts. Journal of Physiology, v.224, n.1, p. 231-244, 1972; Williams, P.E.; Goldspink, G. Changes in sarcomere length and physiological properties in immobilized muscle. Journal of Anatomy, v.127, n.3, p. 459-468, 1978).These alterations are not resulting from disuse, because they are also verified, even faster, in muscles maintainedin shortened position and submitted to electrical stimulation. Thus, in the absence of immobilization, the continuous muscular contraction in shortened position, maintained by electrical stimulation, also results in sarcomere loss (Tabary, J.C. et al. Experimental rapid sarcomere loss with concomitant hypoextensibility, Muscle Nerve, v.4, n.3, p. 198-203, 1981). On the other hand, when the muscle is maintained in the elongated position, there is an increase of up to 19% in the number of sarcomeres in series and an increase in muscle length (Williams, P.E. et al. The importance of stretch and contractile activity in the prevention of connective tissue accumulation in muscle. Journal of Anatomy, v. 158, p. 109-114, 1988; Tabary, J.C. et al. Physiological and structural changes in the cat's soleus muscle due to immobilization at different lengths by plaster casts. Journal of Physiology, v. 224, n.1, p. 231-244, 1972).

Maintenance of muscles in shortened or elongated lengths causes displacements in the length-tension curve, so that the muscles start to generate maximum tension in lengths and joint amplitudes close to those in which they are maintained. Muscles maintained in shortened lengths show a shift of the length-tension curve to the left (muscle starts to generate maximum tension in smaller lengths) and those maintained in elongated lengths show a displacement of the length-tension curve to the right (maximum tension generation in amplitudes of greater length of muscle). (Williams, P.E.; Goldspink, G. Changes in sarcomere length and physiological properties in immobilized muscle. J. of Anatomy, v. 127, n.3, p. 459-468, 1978; Williams, P.E.; Goldspink, G. Changes in sarcomere length and physiological properties in immobilized muscle. J. of Anatomy, v. 127, n.3, p. 459-468, 1978).

Therefore, these evidences suggest that it is possible to change the capacity of force generation and the mechanical resistance offered by muscles to joint movement, through a muscular training in specific joint positions and muscular lengths.

In addition to the evidences obtained in animal models, studies carried out with human beings have demonstrated that resistance exercises performed prioritizing joint amplitudes in which the muscles are elongated or shortened modify the capacity of the individual to generate force in the trained amplitude. It was demonstrated that this training resulted in tissue remodeling (displacement of the length-tension relationship), besides modifications in the joint stiffness. Both effects are consistent with changes in the muscle length generated by changes in the number of sarcomeres in series. This training was effective not only in healthy individuals, but also in children with cerebral palsy (CP). However, the modifications in muscle properties alone did not result in changes in these children's functionality. A possible explanation is that the training intensity has not been enough. In spite of the gains, the imbalance in tissue stiffness still remains. These imbalances generate a tension gradient, in the musculoskeletal system, which facilitates the atypical pattern of movement, since the body segments tend to move in the direction of lower resistance. However, other factors may be associated with the absence of training effects on functional capacity (Aquino, C.F., Fonseca, S.T., Gonçalves, G.P., Silva, P.L., Ocarino, J.M., Mancini, M.C. Stretching versus strength training in lengthened position in subjects with tight hamstring muscles: A randomized controlled trial. Manual Therapy, v.15, p.26-31, 2010; Ocarino, J., Fonseca, S.T., Silva, P.L., Mancini, M.C., Gonçalves, G. Alterations of stiffness and resting position of the elbow joint following resistance training. Manual Therapy, v.13, p.411-418, 2010, Vaz, D.V., Mancini, M.C., Fonseca, S.T., Vieira, D.S., Pertence, A.E.M. Muscle stiffness and strength and their relation to hand function in children with hemiplegic cerebral palsy. Developmental Med. and Child Neurology, v.48, n.9, p.728-733, 2006).

Contemporary theories of motor learning suggest that gains are specific to the type of trained movement. In the case of the study discussed above, the children were requested to simply perform isolated movements of flexion and extension of wrist. This gain was not transferred to manual activities that require, in addition to postural stability, movement of the whole upper limb and coordination of this movement with objects in the environment. Therefore, the minimization of deficiencies in the muscle structure, although necessary, is not enough to cause significant changes in the movement patterns that support the functional activities. In fact, linear and clear relationships among deficiencies in structures and body functions are rarely observed. For the gains to be transferred to performance of daily activities, specific functional trainings should be added to the intervention (Carr, J.H., Shepherd, R.B. Neurological rehabilitation: optimizing motor performance, p.350. Oxford, UK: Butterworth-Heinemann, 1998; Latash ML, Anson JG. What are "normal movements" in atypical populations? Behavioral and Brain Sciences, v.19, n.1, p. 55-106, 1996).

A support for this hypothesis was the study carried out by researchers who evaluated the effects of the constraint-induced movement therapy in the use of the affected upper limb of hemiplegic children. At first, the non-affected upper limb was constraint. During the constraint period, an intensive training was conducted to provide the child countless opportunities to perform activities with the affected limb. Soon after, a bimanual training was implemented. It was observed that this intervention resulted in significant gains in the child's functional performance. Considering, however, that atypical movements are adaptive, it is expected that the effects of the functional training are potentiated, and more easily maintained, if it is associated with interventions aimed at modifying the muscle intrinsic properties that seem to perpetuate the postural and movement compensations associated with CP. (Brandão M.B. et. al. Adapted version of constraint-induced movement therapy promotes functioning in children with cerebral palsy: a randomized controlled trial. Clin Rehabil. v. 24, p.639-47, 2010).

The above-mentioned evidences suggest that effective interventions to promote postural and movement optimization in the context of rehabilitation interventions, in sports and occupational activities should be ruled in two pillars:
I. Intensive strength training, in specific positions, to modify the muscle intrinsic properties in order to (a) promote muscle strength gain in joint amplitudes important to perform functional activities; and (b) optimize the tension distribution, reducing the resistance to movement patterns that guarantee a better performance in these activities.
II. Functional training which propitiates the exploration of the musculoskeletal resources obtained with the strength training in the context of relevant activities.

Some patent documents, concerning the use of vests used in the treatment of motor dysfunctions, are described technically.

The patent document US20070135278, entitled "Suit for forcedly modifying a human posture and producing an increased load on a locomotion apparatus" describes a device that is used to modify the human posture and to produce an increase of load. The device comprises supports for shoulder, pelvis, knee and foot which are connected to each other. The vest that covers the whole chest is interconnected to the support of shoulders and a short through buckles. This device is destined to the treatment of neurological, orthopedic and muscular diseases.

The patent request US20070004570, entitled "Device for treatment of patients with disturbed posture and motor activity" shows a device that comprises a vest, pants and support for knees constituted by non elastic material interconnected by elastic bands.

The patent document WO2008144078, entitled "Neurological motor therapy suit" refers to a device that is constituted by non elastic and removable material. This device is composed by a vest which completely surrounds the upper trunk, and by pants that extends around the hips and thigh of the patient. These parts are interconnected by elastic bands. Optionally, there are supports for elbow, knee, head, hands and feet that are also interconnected with elastic straps.

In the market, there are vests created only for postural support of individuals with motor dysfunctions. These are based on a different theoretical conception of the present invention and they are used in Europe, United States and Brazil.

One of the models on the market, denominated "Therasuit", is used in the treatment of CP. It consists of a soft, proprioceptive and dynamic orthosis, which is constituted by: cap, a suit (made up of short and a vest), knee pads and connections with tennis. All components are connected to each other by a system of elastic strings. The method involves a typical intensive exercise program performed from 3 to 4 hours a day, 5 days a week, during 3 or 4 weeks (Available in: http://www.suittherapy.com; Accessed on: 10/27/2011).

The vest "Adeli" is a device used for treatment of individuals with CP, which uses loads for correction of posture and movement. It consists of a suit composed by vest, shorts, knee pad and shoes. These devices are interconnected by special elastic bands, which are positioned to work as antagonistic muscles and adjusted according to the individual's need, promoting controlled resistance by exercising several muscle groups (Available in: http://www.trajeadeli.com; Accessed on: 10/27/2011).

In particular, the vests available in the market do not explore the geodesic organization principles and tension distribution inherent to the musculoskeletal system, when understood as a tensegrity structure. The elastic structures that compose the vests available in the market do not show interconnections which ensure global distribution of tension or omnidirectional stability. Besides, the absence of prestress in the elastic elements of the vest already marketed requires high local stiffness of these elements to control undesirable movements, which turns these vests excessively restrictive. Finally, the non-geodesic organization of the elastic bands results in displacement of them during the movement. This displacement may modify the action of the elastic bands on the joints so that they harm or hinder important movements for the desired action.

The present matter, on the other hand, is composed by a biomechanical vest, based on tensegrity, which possesses areas reinforced by prestressed materials that simulate the tension of body structures and have a pattern of geodesic organization and continuity throughout the vest.

### FIGURES DESCRIPTION

**Figure 1** displays, in non-limiting manner, the base clothing of the biomechanical vest (front view).
**Figure 2** displays, in non-limiting manner, the base clothing of the biomechanical vest (rear view).
**Figure 3** displays, in non-limiting manner, the base clothing of the biomechanical vest (oblique view).
**Figure 4** displays, in non-limiting manner, the topographical distribution of anchors (front view).
**Figure 5** displays, in non-limiting manner, the topographical distribution of anchors (rear view).
**Figure 6** displays, in non-limiting manner, the topographical distribution of anchors (oblique view).
**Figure 7** displays, in non-limiting manner, the distribution of traction elements (front view).
**Figure 8** displays, in non-limiting manner, the distribution of traction elements (rear view).
**Figure 9** displays, in non-limiting manner, the distribution of traction elements (oblique view).
**Figure 10** displays, in non-limiting manner, the mold vest with traction elements directly attached (fused) on the clothing.
**Figure 11** displays, in non-limiting manner, the mold vest with traction elements directly attached (fused) on base clothing and its connections with the anchors.
**Figure 12** displays, in non-limiting manner, the topographical distribution of the anchors and traction elements of the hand accessory (anterior and posterior views).
**Figure 13** displays, in non-limiting manner, the topographical distribution of the anchors and traction elements of the feet accessory (front, side and rear views).
**Figure 14** displays the graph that shows the obtained results (mean and standard error) with application of the vest for reduction of shoulder protrusion.
**Figure 15** displays the graph that shows the obtained results (mean and standard error) with application of the vest to reduce hip medial rotation and to increase knee lateral rotation during unipodal squat.

### DETAILED DESCRIPTION OF THE INVENTION

The present invention comprises biomechanical vests for stabilization and optimization of posture and movements, enabling interventions based on principles of tissue remodeling and motor learning. The equipment consists of a vest that has areas reinforced by a material that simulates the elastic traction, usually provided by body structures. These areas of tensional reinforcement follow the architecture patterns of the musculoskeletal system and aim to give proper support for posture and movements of children and adults with motor dysfunctions, as well as optimizing the performance of workers and athletes during their activities.

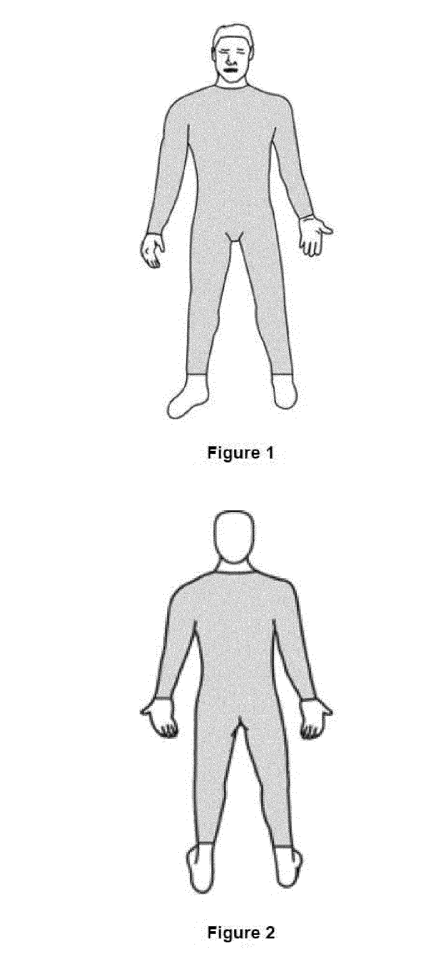

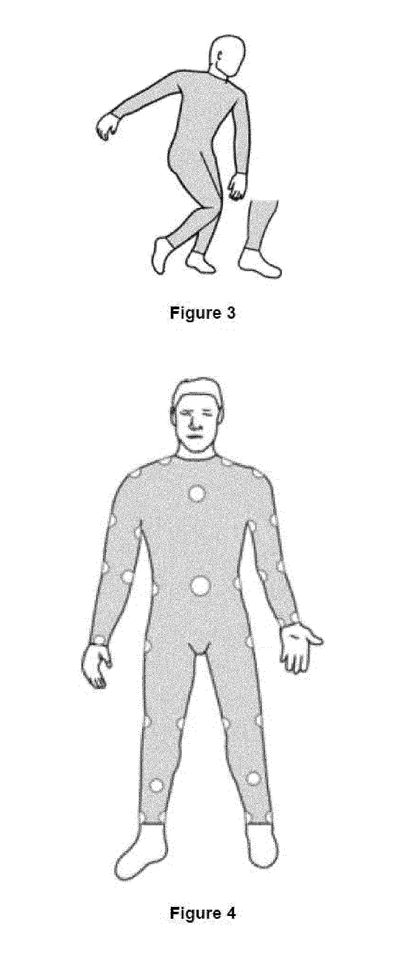

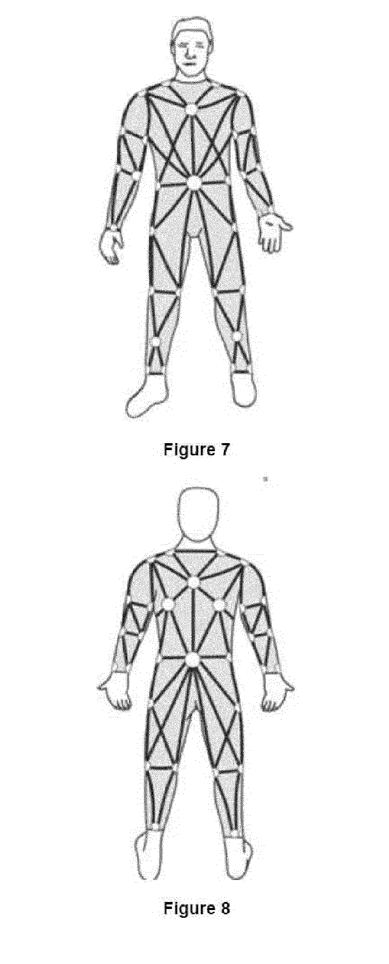

The tensegrity vest is composed by a base clothing (Figures 1, 2 and 3) and accessories, which are wrapped in traction elements (Figures 7 to 11) interconnected and anchored to supporting units (anchors) positioned in strategic points of the body (e.g. trunk, pelvis, upper and lower limbs). The specific positioning of the traction elements (Figures 7 to 11) and of its anchors (Figures 4 to 11) creates force transmission paths similar to those already identified in the musculoskeletal system. (Myers, T.W.; The 'anatomy trains'. Journal of Bodywork and Movement Therapies, v.1, n.2, p.91-101, 1996. Myers, T.W. The 'anatomy trains': part II. Journal of Bodywork and Movement Therapies, v.1, n.3, p.134-145, 1997).

The base clothing is composed by a fair jumpsuit which covers the whole body, except for feet, hands and head (Figures 1, 2 and 3) . This jumpsuit is made with a fabric that has good adherence to the body, without restricting the movement of the joints. Moreover, the fabric does not hinder transpiration and can be used with comfort in all seasons of the year. All materials that possess these characteristics, such as suplex, mesh, polyester, polyamide, among other, are used for making the base clothing.

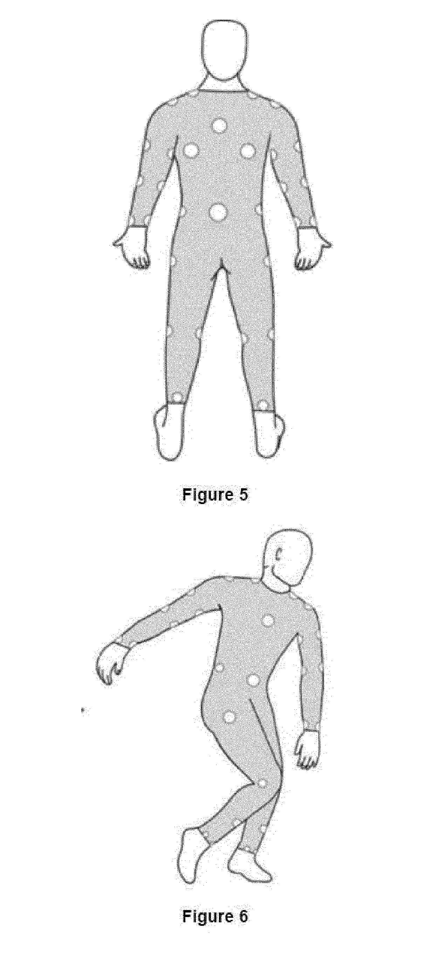

Anchors for elastic elements are structures that contain attachment points for the traction elements (Figures 7 to 11) of the vest. These anchors have a role similar to the nodes of tensegrity structures, guaranteeing the continuity among the traction elements (Figures 7 to 11) and allowing tension distribution throughout the vest. These fixation points have an adjustment mechanism for the tension of the traction elements (Figures 7 to 11), allowing the adjustment of the tension distribution pattern according to the user's specific needs. An implementation of the topographical distribution of the anchors in the front of the vest can be visualized in Figure 4, and in the rear part, in Figure 5. Figure 6 displays an oblique view of the anchors.

Anchors are made with material that fits to the body shape where they are positioned. Besides, the anchor's material has stiffness enough to impede its deformation under the forces exercised by the traction elements connected to them. Any material that has these characteristics, such as leather and canvas, can be used to make the anchors.

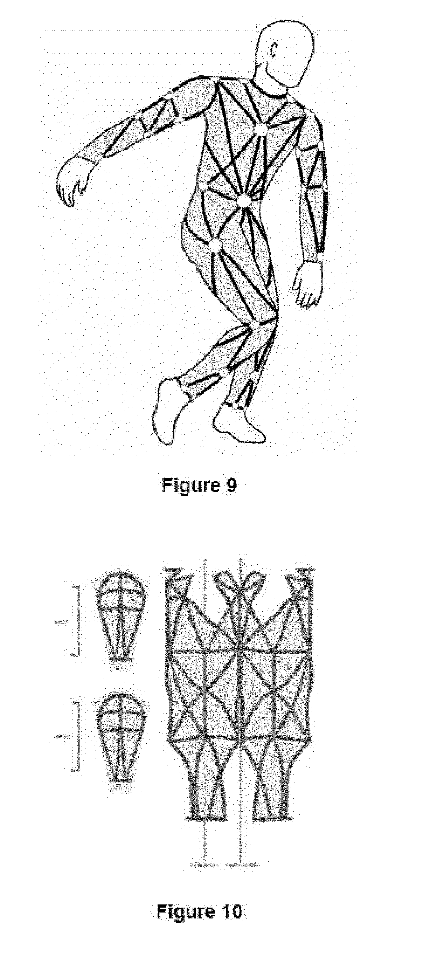

The narrow elastic or viscoelastic bands connect two anchors, following the shortest path among them (geodesic organization) considering the topography of body surface. This geodesic organization of the traction elements (Figures 7 to 9) of the vest is similar to the distribution of traction elements of tensegrity structures. Furthermore, the geometric organization of these elements follows a triangular distribution that ensures self-stabilizing behavior and omnidirectional tension distribution. Each traction element of the vest is balanced by other traction elements (Figures 7 to 9) that offer opposite and continuous tension. Besides, these elements are also balanced by a compression element that pushes the traction elements and anchors. In the context of the vest, this compression element is the user's body. An implementation of a topographical distribution of the traction elements (Figures 7 to 9) at the front of the vest can be seen in Figure 7 and, at the posterior part, in Figure 8. Figure 9 displays an oblique view of the traction elements (Figures 7 to 9). Traction elements (Figures 7 to 9) may be attached only to the anchors, as described previously, which allows them to slide on the base clothing and the tension of these elements is adjusted according to the user's needs and therapeutics.

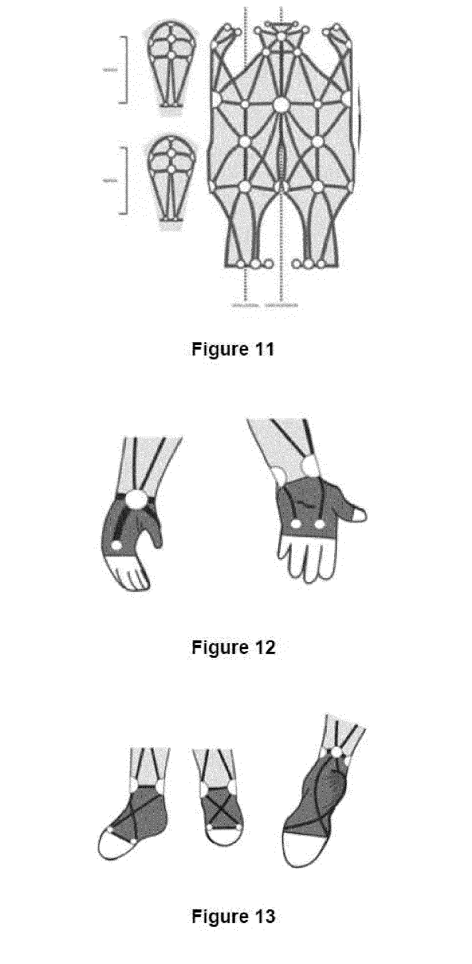

Another possibility is that these elements are attached directly (fused) on the base clothing, while maintaining their connections with the anchors, as shown in Figure 10 and 11.

Traction elements (Figures 7 to 11) of the vest are made with elastic materials that produce tension depending on the magnitude of deformation (the greater the deformation, the greater the resistance produced). These elements may have or not a viscous component which modifies its response according to the deformation speed (the faster, the greater the resistance produced). Moreover, the rate of tension increase of the traction elements (Figures 7 to 11) in response to their deformation should be non-linear from the beginning of this deformation. These elements will offer low resistance to small deformations and gradually increased resistance to higher deformations. These mechanical properties aim to offer resistance to movement in specific directions to be defined according to the user's needs, allowing freedom of movement at the same time. Any material that has these characteristics may be used for making the traction elements (Figures 7 to 11) such as polyurethane, latex, elastic fabrics, among others.

The vest also includes accessories, such as hand gloves and socks; made with the same material of the base clothing. The glove covers the hand until the proximal phalanx of all fingers (Figure 12) and contains anchors for traction elements (Figures 7 to 13) at the metacarpal heads level. The socks cover the whole foot (Figure 13) and contain anchors for traction elements (Figures 7 to 13) at the metatarsals level.

The proposed vest, due to contemplating the properties of prestress, interconnection and geodesic geometry, favors the global and immediate distribution of internal and external forces involved in the body movement. This organization provides an ideal support for dynamic coordination between body segments, necessary to perform functional activities. Specifically, the vest provides an external structure complementary to the musculoskeletal system with dynamically adjustable tension to favor the emergence of postures and movements which optimize the functional performance, and protect the tissues and joints against mechanical stresses associated with the development of pathological conditions.

The proposed vest is based on the understanding of the organization and functioning of the musculoskeletal system as a tensegrity structure. Three fundamental characteristics of tensegrity structures are present in the vest: prestress, interconnection and geodesic organization.

The prestress, present in traction elements (Figures 7 to 11), ensures some stress level (or tension) in all these elements and in all positions assumed by the individual. This property also guarantees immediate responses to mechanical disturbances, aiding in the process of stabilization and optimization of posture and movement and allows control without need of high local stiffness, ensuring flexibility.

The interconnection of the vest is ensured by the connection between all traction elements (Figures 7 to 11) through anchors (connectors) distributed along the vest. This property guarantees the interaction between all body segments and, associated with prestress, enables the global distribution of stresses throughout the body.

The paths delineated by traction elements (Figures 7 to 11) between anchor pairs follow geodesic lines, in other words, the shortest distance among two points on a curved surface. In structures that follow geodesic organization, the traction elements (Figures 7 to 11) are not collinear, instead, they form triangles along all vest ensuring multidirectional stabilization of posture and movement.

Postures and movements, although favored by the vest, are performed actively by the user, involving activation of muscles, in specific positions. This pattern of muscle use leads to adaptations of the properties of biological tissues, which favor the implementation of postures and movements facilitated by the vest, creating a virtuous cycle.

The performance improvement using the vest is provided by: changes in the capacity of active and passive generation of tissue tension in ranges of motion relevant to the performance of functional activities; optimizing the tension distribution, in order to reduce the resistance to movement patterns that guarantee a better performance in these activities; and allowing continuous functional training that permit the individual to learn to use, during important activities, the developed neuromusculoskeletal capabilities. The discussed matter is a tool that provides support to rehabilitation programs, aimed at individuals with movement and posture dysfunctions, and training programs for improving sports performance and occupational activities. The vest provides postural support so that the muscles can be exercised in functional positions, in order to favor the tissue remodeling associated with the increase of active and passive generation of tissue tension necessary for performance improvement. This tissue remodeling promotes a great distribution of tension, in order to reduce the resistance to movement patterns that ensure a better performance in these activities. Furthermore, the use of the vest allows the accomplishment of a continuous functional training that enables the individual to learn to use, during important activities, the developed neuromusculoskeletal capabilities. Finally, the continued use of the vest allows that the gains obtained in the training are maintained, since the muscles are exercised in target lengths and joint positions, in the context of day-to-day activities.

The tensegrity vest encompasses means to optimize the performance of workers and athletes.

The present technology can be better understood through the following, but not limiting, examples.

### EXAMPLE 1 - RESULTS OF STUDIES DEVELOPED WITH THE TECHNOLOGY FOR OPTIMIZATION OF POSTURE

A study was carried out to investigate whether the use of the present vest, with the tension increase of the traction elements posterior to the scapulas, is capable to reduce the posture of shoulder profusion. A relaxed posture of shoulder profusion was measured with a three-dimensional movement analysis system (Codamotion, Charnwood Dynamics Ltd., Rothley, England), in the Movement Analysis Laboratory of the School of Physical Education, Physical Therapy and Occupational Therapy of Universidade Federal de Minas Gerais (UFMG). These measures were performed during the use of the vest, in 10 healthy adult volunteers. For each volunteer, the following measures were performed: (a) measures using the vest without manipulations of tensions of the traction elements (elastic) and (b) measures using the vest with tension increase of the traction elements posterior to the scapulas. The volunteers were in standing position, more relaxed as possible and were not informed on the expected effect of the manipulation of the traction elements. Three measures were carried out in each situation (pre and post tension manipulation of the traction components) and the means of these measures were statically analyzed using paired t-tests with significance level of 0.05.

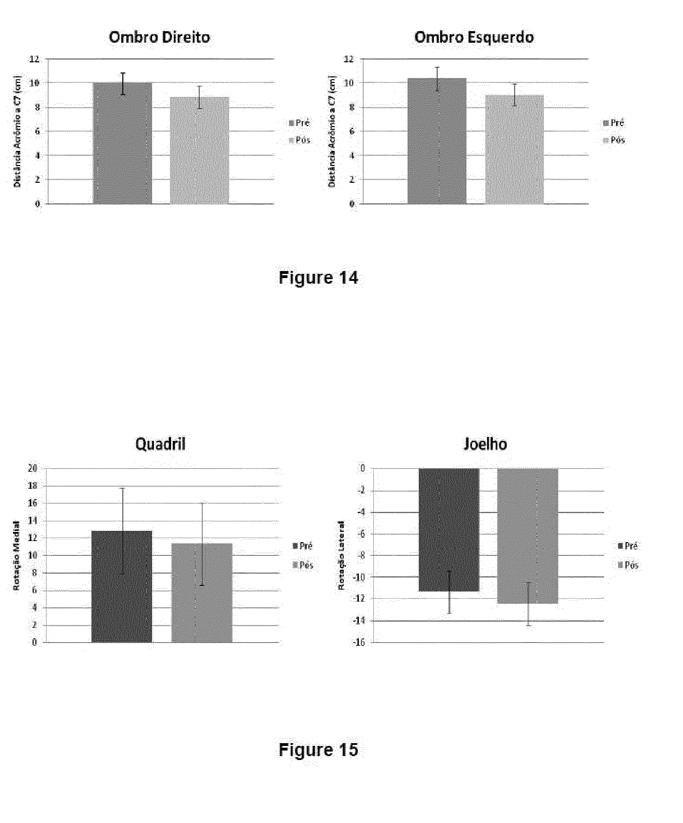

Tension manipulation of specific traction elements of the vest generated a statistically significant reduction in shoulder profusion, as hypothesized, in both the right shoulder (p <0.001) and the left shoulder (p <0.001) (Figure 14).

This postural effect is considered clinically important, since excessive shoulder protrusion may lead to the development of orthopedic pathological processes. (LUDEWIG, P.M.; REYNOLDS, J.F. The association of scapular kinematics and glenohumeral joint pathologies. J Orthop Sports Phys Ther, v. 39, n.2, p. 90-104, Feb. 2009).

According to the results showed in Figure 1, the use of the vest, adjusted to obtain this effect (condition "post"), led to a significant reduction of the distance between the seventh cervical vertebra and the anterior border of acromion, in the sagittal plane, compared with the vest without these fittings (condition "pre"), which demonstrates reduction of shoulder protrusion. This proves that the vest is capable to change posture components of clinically importance for rehabilitation.

### EXAMPLE 2 - RESULTS OF STUDIES DEVELOPED WITH THE TECHNOLOGY FOR OPTIMIZATION OF HUMAN MOVEMENT

A study was carried out to investigate whether the use of the vest, with tension increases in the traction components posterior to the hip (which may generate a lateral rotation torque at this joint) is capable to reduce the movement of hip medial rotation and to increase the movement of knee lateral rotation. This study was performed with the same equipments and volunteers of the study demonstrated as example 1. For each volunteer, the following measurements were performed: (a) measures using the vest without tension manipulations of the traction elements and (b) measures using the vest with tension increases of the traction elements posterior to the hip, which may generate a lateral rotation torque at the hip. The volunteers performed unilateral squats (with only one lower limb on the ground), with a maximum knee flexion of 60 degrees. The volunteers were not informed on the expected effects of the manipulation of the traction elements. Three measures were carried out in each situation (pre and post tension manipulation of the traction components) and the means of these measures were statistically analyzed using paired t-tests with significance level of 0.05.

Tension manipulation of specific traction elements of the vest generated a statistically significant reduction of the hip medial rotation (p=0.043) and a statistically significant increase of knee lateral rotation (p=0.043) (Figure 15), as hypothesized. This effect is considered clinically important, since the excessive hip and knee medial rotation movements are related to neurological and orthopedic dysfunctions and are commonly targets of clinical interventions (POWERS, C.M. The influence of abnormal hip mechanics on knee injury: a biomechanical perspective. J Orthop Sports Phys Ther, v. 40, n.2, p. 42-51, Feb. 2010).

According to results shown in Figure 15 the use of the vest, adjusted to obtain these effects (condition "post"), led to a significant reduction of the hip medial rotation and a significant increase of knee lateral rotation, compared with the vest without these fittings (condition "pre"). This proves that the vest is capable to change movement components of clinically importance for rehabilitation. Further, the vest is capable to generate effects distant of the manipulation, in agreement with the proposal of the vest, which was revealed by the effects in the knee joint resulting from tension manipulation of the traction components at the hip.

## Claims

1. Tensegrity vest, **characterized by** comprising the base clothing (Figures 1, 2 and 3); anchors (Figures 4, 5 and 6); traction elements (Figures 7, 8, 9, 10 and 11) and, optionally, accessories for hands and feet (Figures 12 and 13).

2. Tensegrity vest, according to claim 1, **characterized by** the anchors are the fixing points for traction elements.

3. Tensegrity vest, according to claims 1 and 2, **characterized by** the anchors allowing adjustments of traction elements.

4. Vests based on tensegrity, according to claims 1, 2 and 3 **characterized by** traction elements connecting two anchors, following the shorter path between them and having a triangular distribution.

5. Tensegrity vest, according to claims 1, 2 and 4, **characterized by** traction elements can be attached directly (fused) on the base clothing, still maintaining their connections with their anchors as established in figures 10 and 11.

6. Tensegrity vest, according to claim 1, **characterized by** material of base clothing and accessories belonging, preferably, to the group comprising cotton, suplex, knitted, polyester and polyamide.

7. Tensegrity vest, according to claim 1, **characterized by** material of anchors belonging, preferably, to the group comprising synthetic leather, plastics, leather e canvas.

8. Tensegrity vest, according to claims 1, 4 and 5 **characterized by** material of traction elements belonging to the group comprising polyurethane, latex, natural and synthetic fabrics with elastic behavior, and containing or not viscous materials.

9. Tensegrity vest, according to claims 1 to 8, **characterized by** promoting posture and movement stabilization and optimization.

10. Tensegrity vest, according to claims 1 to 9, **characterized by** comprising proper support for posture and movement of children and adults, preferably, with motor dysfunctions.

11. Tensegrity vest, according to claims 1 to 10, **characterized by** comprising means to optimize performances of workers and athletes.

## Amended claims

### Amended claims under Art. 19.1 PCT

**1.** Vestment to optimize human posture and movement, wherein it is based on tensegrity, i.e. pre-stress, interconnection and geodetic organization, and comprises traction elements interconnected and anchored to the supporting units positioned in strategic points of the body, said anchoring having fastening points for traction elements and narrow elastic or viscoelastic bands that connect two anchoring.

**2.** Vestment to optimize human posture and movement, according to claim 1, wherein the traction elements connect two anchoring, following the smaller path between them, and present triangular distribution.

**3.** Vestment to optimize human posture and movement, according to claim 1, wherein the anchoring are manufactured with elasticity material limited that allows accommodating to the body region shape where they are positioned to produce tension in function of the deformation magnitude.

**4.** Vestment to optimize human posture and movement, according to claim 1, wherein the elastic or viscoelastic bands follow the smaller path between them considering the body surface type.

**5.** Vestment to optimize human posture and movement, according to claim 1, wherein it comprises a base cloth that optionally will be used to fasten the anchoring, gloves that cover the hand and have anchoring for traction elements and socks that have anchoring for traction elements.

**6.** Use of the vestment to optimize human posture and movement, wherein it is used in maintenance of rehabilitation programs towards individuals with movement and posture disorders and in training programs to optimize the performance with sports and occupational activities.

**7.** Use of the vestment to optimize human posture and movement, according to claim 6, wherein it is used in the changes induction in the capacity of active and passive generation of tissue tension in movement amplitudes relevant to the functional activities performance, in the tension distribution optimization and in the continuing functional training performance.

**8.** Use of the vestment to optimize human posture and movement, according to claim 6, wherein it is appropriate for children and adults.
